## Europäisches Patentamt

## European Patent Office

## Office européen des brevets

(19)

(11) Numéro de publication: **0 112 261**
**B1**

(12)

# FASCICULE DE BREVET EUROPÉEN

(45) Date de publication du fascicule du brevet:
20.05.87

(21) Numéro de dépôt: 83420183.2

(22) Date de dépôt: 05.12.83

(51) Int. Cl.⁴: **C 07 C 45/38,** C 07 C 45/39,
C 07 C 45/84, C 07 C 47/02,
C 07 C 47/21, C 07 C 49/04,
C 07 C 49/403, C 07 C 49/17,
B 01 J 23/56

(54) Procédé d'oxydation d'alcools en composés carbonyles correspondants.

(30) Priorité: 08.12.82 FR 8220808

(43) Date de publication de la demande:
27.06.84 Bulletin 84/26

(45) Mention de la délivrance du brevet:
20.05.87 Bulletin 87/21

(84) Etats contractants désignés:
AT BE CH DE FR GB IT LI LU NL SE

(56) Documents cité:
EP-A-0 028 714
EP-A-0 044 260
FR-A-2 305 420
FR-A-2 350 323
US-A-3 673 257
US-A-4 218 401

CHEMICAL ABSTRACTS, vol. 96, no. 13, juin 1982,
page 704, no. 217471w, Columbus, Ohio, US
CHEMICAL ABSTRACTS, vol. 89, 1978, page 579, no.
179705w, Columbus, Ohio, US
CHEMICAL ABSTRACTS, vol. 97, no. 13, décembre
1982, page 795, no. 215552v, Columbus, Ohio, US

Le dossier contient des informations techniques
présentées postérieurement au dépôt de la
demande et ne figurant pas dans le présent
fascicule.

(73) Titulaire: RHONE- POULENC S.A., 25, quai Paul
Doumer, F-92408 Courbevoie (FR)

(72) Inventeur: Costantini, Michel, 9, Place Aristide
Briand, F-69003 Lyon (FR)
Inventeur: Krumenacker, Léon, 2, Allée du Bois
rond, F-69360 Serezin du Rhône (FR)

(74) Mandataire: Vignally, Noel, RHONE- POULENC
INTERSERVICES Service Brevets Chimie Centre
de Recherches de Saint- Fons B.P. 62, F-69192
Saint- Fons Cedex (FR)

EP 0 112 261 B1

## Description

La présente invention concerne la préparation d'aldéhydes ou de cétones par oxydation partielle des alcools.

On connaît certains procédés permettant d'obtenir des aldéhydes ou des cétones par oxydaticn des alcools correspondants.

Ainsi le brevet français n° 75.09932 (publié scus le n° 2 305 420) décrit l'oxydaticn de l'alcool hydroxy-2 benzylique en aldéhyde salicylique par l'oxygène ou l'air, en milieu aqueux alcalin et en présence d'un catalyseur constitué par du platine ou du palladium asscció à un sel de bismuth et déposé sur un support solide.

Ce procédé, très intéressant dans le cas des alcools hhydroxybenzyliques, n'est pas généralisable et lorsqu'il est appliqué par exemple à des alcanols primaires, il conduit à une forte proportion d'acides.

Le brevet français n° 76.24 530 (publié sous le n° 2 325 628) décrit un procédé d'oxydation d'un alcool éthylénique (l'alcool chrysanthémique) en aldéhyde correspondant, par l'oxygène ou l'air, dans un milieu liquide constitué généralement par un hydrocarbure ou un ester d'alkyle inférieur d'acides carboxyliques saturés inférieurs et en présence d'un catalyseur constitué par du platine déposé sur carbone. Il est recommandé d'utiliser au moins 10 % en poids de platine par rapport à l'alcool et d'effectuer la réaction sur des solutions contenant moins de 4 à 5 % d'alcool à oxyder. Ces conditions rendent ce procédé économiquement peu intéressant.

Un article du Journal of American Chemical society de 1955 (volume 77, pages 190-191) décrit une méthode de déshydrogénation catalytique d'alcools primaires et secondaires en aldéhydes et cétones par l'oxygène en présence de platine. La réaction est conduite dans différents solvants dont les plus intéressants sont l'acétate d'éthyle, l'eau et l'acétone diluée; le platine est sous forme de platine en poudre obtenu par réduction de l'oxyde de platine. Les quantités de catalyseur sont, ici également, trop élevées (d'environ 25 à 50 % en poids de platine métal par rapport à l'alcool à déshydrogéner) pour qu'un tel procédé soit économiquement utilisable. En outre les durées de réaction sont généralement très longues (de l'ordre de 10 à 24 heures) malgré ces quantités prohibitives de catalyseur.

Le brevet européen EP 44.260 décrit la préparation de polyphénols par une succession d'étapes. L'une de ces étapes consiste à oxyder par l'oxygène un dihydroxyméthyl gaïacolate alcalin, en solution aqueuse, en présence d'un catalyseur à base de platine déposé sur un support inerte et contenant éventuellement du Cd, Ce, Bi, Ag, Te ou Sn, entre 30° et 60°C, le pH du milieu étant maintenu entre 11,5 et 12.

Ce procédé présente l'inconvénient de nécessiter un milieu très alcalin.

On ne dispose donc actuellement pas de procédé général et efficace d'oxydation sélective d'alcools primaires ou secondaires en aldéhydes ou cétones correspondants, qui puisse être utilisé industriellement.

L'objet de la présente invention est de fournir un tel procédé. Plus précisément l'invention consiste en un procédé de préparation d'aldéhydes ou de cétones par oxydation catalytique en milieu liquide des alcools primaires ou secondaires correspondants, à l'aide d''oxygène moléculaire ou d'un gaz en contenant, caractérisé en ce que:

- le catalyseur est constitué par un métal noble du groupe VIII de la classification periodique des éléments associé à un métal, choisi dans le groupe comprenant le bismuth, le plomb, le cadmium, le mercure, l'indium, le tellure, l'étain, l'argent et leurs dérivés, déposés sur un même support solide,
- le milieu liquide est constitué par un solvant organique,
- la température de réaction est comprise entre 60°C et 180°C,
- l'on élimine l'eau formée.

Les alcools auxquels s'applique le procédé selon l'invention peuvent être tous les alcools primaires ou secondaires, mono- ou polyfonctionnels, ayant de 2 à 36 atomes de carbone, aliphatiques saturés ou comportant une ou plusieurs insaturations éthyléniques ou acétyléniques, cycloaliphatiques saturés ou comportant une ou plusieurs insaturations éthyléniques, arylaliphatiques, hétérocycliques, ou tels que leur formule comporte un enchaînement de plusieurs radicaux choisis parmi les radicaux aliphatiques, cyclcaliphatiques, aromatiques ou hétérocycliques.

Plus précisément ces alcools répondent à la formule générale (I):

$R_2$ - CHOH - $R_1$ (I)

dans laquelle:

- $R_1$ et $R_2$, identiques ou différents, représentent:

+ un radical alkyle linéaire ou ramifié ayant de 1 à 35 atomes de carbone, tel que par exemple un radical méthyle, éthyle, propyle, butyle, pentyle, hexyle, octyle, décyle, dodécyle, hexadécyle, octadécyle, eicosyle,

+ un radical alkényle linéaire ou ramifié ayant de 2 à 35 atomes de carbone, tel que par exemple un radical vinyle, allyle, buténogène, pentényle, hexényle, octényle, décényle, dodécényle, octadécényle,

+ un radical alkynyle, linéaire ou ramifié, ayant de 2 à 35 atomes de carbone tel que par exemple un radical éthynyle, propyne-2 yle, pentynyle, hexynyle, octynyle, décynyle, dodécynyle, tétradécynyle, octadécynyle, tétracosynyle;

+ un radical alkadiényle ou alkadiynyle linéaire ou ramifié, ayant de 4 à 35 atomes de carbone tel que par exemple un radical heptadièna-1,5 yle ·

+ un radical cycloaliphatique ayant de 3 à 12 atomes de carbone et pouvant comporter 1 ou plusieurs insaturations éthyléniques, tel que par exemple un radical cycloprcpyle, cyclobutyle, cyclopentyle, cyclohexyle, cyclooctyle, cyclodécyle, cyclododécyle, cyclohexène-2 yle, cyclohexène-3 yle, cyclopentène-2 yle, cyclopentène-3 yle, cyclooctène-3 yle, cyclodécène-4 yle, cyclohexadiène-2,5 yle ou cyclopentadiène-2,4 yle,

+ un radical aryle tel que par exemple un radical phényle ou naphtyle; les radicaux aliphatiques indiqués précédemment pouvant comporter un ou plusieurs atomes d'oxygène - O - ou groupements - CO - et les radicaux cycloaliphatiques pouvant comporter un ou plusieurs groupements - CO -

+ un radical hétérocyclique saturé ou insaturé comportant de 3 à 12 atomes de carbone et 1 ou plusieurs hétéroatomes comme l'oxygène, le soufre et l'azote, tel que par exemple un radical thiényle, furyle, pyrannyle, pyrrolyle, pyramolyle, pyridyle, pyrazinyle, pyrimidinyle, pyridasinyle, isisothiazolyle, iaoxazolyle, furazannyle, pyrrolidinyle, imidazolidinyle, pyrazolidinyle, delta 3-pyrazolinyle, delta 2-pyrrolinyle, delta 2 imidazolinyle, pipéridyle, morpholinyle, triazinyle, azocinyle, thiazolyle ou oxathiolannyle;

les différents radicaux aliphatiques, cycloaliphatiques, aromatiques ou hétérocycliques indiqués précédemment pouvant comporter un ou plusieurs substituants tels que groupement hydroxyle, groupement alkoxy ayant de 1 à 6 atomes de carbone, groupment nitro, groupement nitrile ou atome d'halogène;

- $R_1$ ou $R_2$ représente un atome d'hydrogène

- $R_1$ et $R_2$ peuvent former ensemble un radical alkylène, ou alkénylène, ou alkadiénylène ou alkynylène ou alkadiynylène, linéaire ou ramifié, ayant ou non un ou plusieurs substituants tels que groupement hydroxyle, groupement alkoxy ayant de 1 à 6 atomes de carbone, groupement nitro, groupement nitrile ou atome d'halogène,

- $R_1$ et $R_2$, isolément ou ensemble, sont constitués par un enchaînement de plusieurs radicaux choisis parmi les radicaux aliphatiques, cycloaliphatiques, aromatiques et hétérocycliques indiqués précédemment;

- $R_1$ et $R_2$ sont tels que la formule (I) comporte au plus 36 atomes de carbone.

A titre d'exemples non limitatifs d'alcools de formule (I) auxquels s'applique le procédé selon l'invention on peut citer notamment:

- des alcools aliphatiques tels que l'éthanol, le propanol-1, le propanol-2, le butanol-1, le butanol-2, le pentanol-1, le pentanol-2, le pentanol-3, l'hexanol-1, l'hexanol-2, l'hexanol-3, l'heptanol-1, l'heptanol-2, l'octanol-1, l'octanol-2, l'cotanol-3, le nonanol-1, le décanol-1, le décanol-2, le dodécanol-1, le dodécanol-2, le tétradécanol-1, le tétradécanol-2, l'hexadécanol-1, l'hexadécanol-2, le docosanol-1, l'alcool allylique, l'oxa-3 pentanediol-1,5, le dioxa-3,6 heptanol-1, le propanediol-1,2, le butanediol-1,4, le diméthyl-3,7 octène-6 ol-1, l'éthyl-3 méthyl-7 octadiène-2,6 ol-1, le pentyl-3 méthyl-7 octadiène-2,6 ol-1, le pentaméthyl-2,3,4,5,7 octadiène-2,6 ol-1, le triméthyl-3,5,7 octadiène-2,6 ol-1, le triméthyl-2,4,7 isopropyl-3 octadiène-2,6 ol-1, le géraniol, l'octadécène-9 ol-1, l'octyne-2 ol-1, le diméthyl-3,7 octanol-2, le méthyl-3 butène-2 ol-1, le méthyl-3 butène-3 ol-1, l'éthyl-2 butène-2 ol-1,

- des alcools arylaliphatiques tels que l'alcool benzylique, l'alcool phénéthylique, l'alcool salicylique, l'alcool hydroxy-4 benzylique, l'alcool méthyl-3 hydroxy-2 benzylique, l'alcool méthyl-3 hydrcxy-4 benzylique, le phényl-3 propène-2 ol-1, le (naphtyl-2)-2 éthanol,

- des alcools oycloalipdatiques, cycloalkylaliphatiques, cycloalkénylaliphatiares tels que le cyclohexanol, le méthyl-4 cyclohexanol, le tertiobutyl-4 cyclohexanol, le chloro-4 cyclohexanol, l'éthyl-2 cyclohexanol, le (méthyl-2 propène-1) yle-2 diméthyl-3,3 cyclopropyl-méthanol, le cyclopentanol, le cycloheptanol, le cyclohexène-2 ol-1, le menthol, le (triméthyl-2,2, 6 cyclohexyl) méthanol, le diméthyl-3,7 (triméthyl-2,6,6 cyclohexène-1 yle)-9 nonatétraène-2,4,6,8 ol-1 (vitamine A).

Parmi les alcools auxquels il s'applique, le procédé selon l'invention, convient tout particulièrement à ceux qui répondent à la formule générale (I) dans laquelle:

- $R_1$ et $R_2$, identiques ou différents, représentent:

+ un radical alkyle linéaire ou ramifié ayant de 1 à 23 atomes de carbone:

+ un radical alkényle ou alkynyle linéaire ou ramifié ayant de 3 à 23 atomes de carbone, et dont la double ou la triple liaison n'est pas située sur l'atome de carbone directement lié à l'atome de carbone portant la fonction OH,

+ un radical alkadiényle ou alkadiynyle, linéaire ou ramifié, ayant de 5 à 23 atomes de carbone et dont l'atome de carbone directement lié à 1 atome de carbone portant la fonction OH ne comporte pas de liaison multiple;

+ un radical cycloaliphatique ayant de 3 à 12 atomes de carbone

+ un radical phénylalkyle dont la partie alkyle comporte 1 à 14 atomes de carbone;

les différents radicaux indiqués précedemment pouvant comporter un ou plusieurs substituants tels que groupement hydroxyle groupement alkoxy ayant de 1 à 4 atomes de carbone groupement nitro, groupement nitrile ou atome d halogène,

- $R_1$ ou $R_2$ représente un atome d'hydrogène

- $R_1$ et $R_2$ peuvent former ensemble un radical alkylène linéaire ou ramifié ayant de 4 à 23 atomes de carbone et ayant ou non un ou plusieuis substituants tels que groupement hydroxyle groupement alkoxy ayant de 1 à 4 atomes de carbone groupement nitro, groupement nitrile ou atome d'halogène,

- $R_1$ et $R_2$, isolément ou ensemble, sont constitués par un enchaînement de plusieurs radicaux choisis parmi les radicaux que peuvent représenter $R_1$ et $R_2$, indiqués précédemment

- $R_1$ et $R_2$ sont tels que la formule (I) comporte au plus 36 atomes de carbone.

Parmi les alcools plue particulièrement définis précédemment la situation des alcools primaires est à considérer plus spécifiquement. En effet pour ces composés le présent procédé apporte outre de bons rendements une solution au délicat problème de l'oxydation sélective en aldéhyde au détriment de l'oxydation

0 112 261

en acide carboxylique.

Ces alcools primaires seront donc notamment ceux qui répondent à la formule gerérale (I) dans laquelle:
- R₁ est un atome d'hydrogène,
- R₂ represente:
+ un radical alkyle linéaire ou ramifié ayant de 1 à 23 atomes de carbone;
+ un radical alkényle ou alkynyle linéaire ou ramifié ayant de 3 à 23 atomes de carbone et dont la double ou la triple liaison n'est pas située sur l'atome de carbone directement lié à l'atome de carbone portant la fonction OH,
+ un radical alkadiényle ou alkadiynyle linéaire ou ramefié, ayant de 5 à 23 atomes de carbone et dont l'atome de carbone directement lié à l'atome de carbone portant la fonction OH ne comporte pas de liaison multiple;
+ un radical cycloaliphatique ayant de 3 à 12 atomes de carbone;
+ un radical phénylalkyle dont la partie alkyle comporte 1 à 14 atomes de carbone;
les différents radicaux indiqués précédemment pouvant comporter un ou plusieurs substituants tels que groupement hydroxyle, groupement alkoxy ayant de 1 à 4 atomes de carbone, groupement nitro groupement nitrile ou atome d'halogène,
- R₂ est constitué par un enchaînement de plusieurs radicaux choisis parmi les radicaux que peut représenter R₂ indiqués précédemment
- R₂ est tel que la formule (I) comporte au plus 36 atomes de carbone.

Le catalyseur utilisé dans le procédé selon l'invention est constitué par l'association:
- d'un metal noble du groupe VIII de la classification périodique des éléments, choisi le plus souvent dans le groupe constitué par le platine, le palladium, le ruthénium et le rhodium,
- et d'un métal qui (sera appelé par commodité cocatalyseur), choisi dans le groupe comprenant le bismuth le plomb, le cadmium, le mercure, l'indium, le tellure, l'étain et l'argent, se trouvant sous forme métallique ou sous forme d'un dérivé organique ou inorganique.

Le métal noble et le cocatalyseur sont déposés sur le même support inerte, qui peut être par exemple du noir de carbone, de la silice de l'alumine, de la silice-alumine, de la magnésie, du sulfate de baryum ou des matières équivalentes.

Le support solide inerte utilisé le plus fréquemment est le noir de carbone.

Le métal noble représente généralement de 0,1 % à 20 % en poids par rapport au poids total métal noblesupport solide.

De préférence ce rapport pondéral est compris entre 0,5 et 10 %.

La quantité de catalyseur utilisée est telle que l'on a généralement un rapport métal noble/alcool variant de 0,01 % à 10 % en poids et de préférence compris entre 0,1 % et 5 % en poids.

Les cocatalyseurs utilises dans le procédé selon l'invention peuvent se trouver au degré d'oxydation 0 ou à l'un des autres degrés d'oxydation qu'ils possèdent.

Pour le dépôt desdits cocatalyseurs sur le support inerte, il est plus pratique pour obtenir une bonne dispersion dans le support d'utiliser un dérivé organique ou inorganique des métaux indiqués précédemment.

A titre d'exemples de tels dérivés on peut citer:
- comme dérivés inorganiques: les oxydes; les hydroxydes; les sels d'hydracides tels que chlorures bromures, iodures, sulfures ou séléniures; les sels d'oxacides tele que sulfites, sulfates, nitrites, nitrates, phosphites, phosphates, pyrophosphates, carbonates, perchlorates, antimoniates, arséniates, sélénites, séléniates, vanadates, niobates, tantalates, chromates, molybdates, tungstates ou permanganates;
- comme dérivés organiques des sels d'acides organiques aliphatiques ou aromatiques tels que acétates, propionates, benzoates, salicylates ou oxalates; des phénolates tels que gallates ou pyrogallates.

Les dérivés qui sont le plus fréquemment utilisés sont les oxydes, les hydroxydes, les nitrates, les sulfates, les halogénures (notamment les chlorures), les phosphates, les carbonates, les acétates, les propionates, les benzoates ou les oxalates.

En pratique le dépôt du cocatalyseur sur le support solide s'effectue selon les méthodes couramment employées. Il peut être par exemple réalisé en mettant le dérivé choisi du métal servant de cocatalyseur en solution, généralement aqueuse et acide, puis en ajoutant à cette solution le support sur lequel le métal noble est déjà présent, enfin en précipitant le cocatalyseur par addition d'un réactif approprié. Le dérivé ainsi déposé peut ensuite être éventuellement réduit, par exemple par le formol, afin que tout ou partie du métal se trouve au degré d'oxydation O.

Une autre méthode courante de préparation du catalyseur consiste à précipiter et fixer sur le support solide simultanément le métal noble et le cocatalyseur, la réduction partielle ou totale des métaux s'effectuant ensuite.

Parmi les métaux pouvant servir de cocatalyseur le bismuth, le plomb et la cadmium conviennent tout particulièrement bien.

Ils permettent notamment une reduction sensible des réactions secondaires.

Le bismuth est plus particulièrement préféré.

La quantite de cocatalyseur exprimée en moles d'atomes du métal utilisé par rapport aux moles d'atomes de métal noble peut varier dans de larges limites. Ce rapport molaire se situe en général entre 0,05 et 10. Il est le plus fréquemment compris entre 0,1 et 2.

Parmi les métaux nobles qui conviennent bien pour la mise en oeuvre du procédé selon l'invention le platine

4

est celui qui est plus spécialement efficace, notamment lorsque le cocatalyseur qui lui est associé est le bismuth.

Le solvant organique constituant le milieu dans lequel s'effectue la réaction de déshydrogénation de l'alcool en aldéhyde ou cétone peut être tout solvant au moins partiel dudit alcool inerte dans les conditions opératoires.

L'eau formée au cours de la réaction de déshydrogénation de l'alcool peut être éliminée par tout moyen adéquat.

Un moyen particulièrement pratique est la distillation azéotropique la température à laquelle s'effectue la réaction est alors celle de l'ébullition du melange réactionnel.

Pour la mise en oeuvre de cette variante préférentielle, il est nécessaire d'utiliser comme solvant organique un liquide formant avec l'eau un azéotrope binaire dont le point d'ébullition est généralement d'au moins 50°C et le plus souvent d'au moins 60°C. Ledit solvant organique est choisi de telle façon que l'azétrope binaire qu'il forme avec l'eau ait un point d'ébullition intérieur:
- au point d'ébullition de l'alcool à déshydrogéner;
- au point d'ébullition de l'azéotrope binaire que ledit alcool serait susceptible de former, soit avec l'eau soit avec le solvant organique lui-même.

Enfin on choisira de préférence le solvant organique de telle façon qu'il ne forme pas d'azéotrope ternaire avec l'alcool à déshydrogéner et l'eau afin de limiter les pertes dudit alcool.

Les solvants organiques pouvant être utilisés dans le cadre du procédé selon l'invention ont généralement un point d'éullition compris entre 50° et 200°C et de préféence entre 60°C et 180°C.

Ils sont habituellement choisis parmi les hydrocarbures aliphatiques cyclcaliphatiques ou aromatiques; les esters d'alkyle ou d'alkényle d'acides carboxyliques aliphatiques; les éthers aliphatiques, aromatiques ou cycliques; les nitriles aliphatiques cycloaliphatiques, ou aromatiques; les cétones aliphatiques, cycloaliphatiques ou aromatiques.

A titre d'exemples non limitatifs on peut citer:
- des hydrocarbures comme le n-hexane, le n-heptane, le n-octane, le n-nonane, le benzène, le styrène, l'éthyl-benzène le toluène, le métaxylène, l'isopropyl-benzène, le cyclohexane, le méthyl-4 pentène-2;
- des esters comme le formiate d'éthyle, le formiate de butyle, le formiate d'isobutyle, l'acétate d'éthyle, l'acétate d'allyle, l'acétate de propyle, l'acétate d'isopropyle, l'acétate de butyle, l'acétate d'hexyle, l'acétate d'heptyle, l'acétate de méthyl-2 propyle, l'acétate d'ethyl-2 butyle, l'acétate de méthoxy-3-butyle, l'acétate de méthyl-4 pentyle-2, l'acétate d'éthyl-2 hexyle, le propionate d'éthyle, le propionate de méthyle, le propionte de vinyle, l'acrylate de butyle, l'acrylate d'éthyle le crotonate d'éthyle, le butyrate d'ethyl-2 butyle, le butyrate de butyle, le butyrate de méthyl-4 pentyle-2, le butyrate de vinyle, l'isobutyrate de vinyle, le méthyl-2 pentanoate de vinyle l'éthyl-2 hexanoate de vinyle le butyrate de méthyle;
- des éthers comme l'éthoxy-1 butène-1 cis, l'éthoxy-1 butène-1 trans, l'oxyde de dibutyle, l'isopropoxy-1 butane, le diméthoxy-1,1 éthane, le diméthoxy-1,2 éthane, le diéthoxy-1,1 méthane, le diméthoxy-1,2 propane, l'éthoxy-1 butane, l'cxyde de diisopropyle, l'éthoxy-1 hexane, l'éthoxy-2 propane, le méthoxy-1 butadiène-1,3 l'oxyde de butyle et de vinyle, l'oxyde d'éthyl-2 hexyle et de vinyle, l'oxyde d'isobutyle et de vinyle, loxyde d'isopropyle et de vinyle, le diméthyl-2,5 furanne;
- des nitriles comme le butyronitrile, l'acrylonitrile, le propionitrile, le tétrahydrobenzonitrile;
- des cétones telles que lacyclopentanone, ladipropylcétone, l'heptanone-3, la méthyl-4 pentène-3 one-2, la méthyl-5 hexanone-2, la pentanone-2.

Comme cela a été insdiqué précédemment la température à laquelle s'effectue la réaction de déshydrogénation des alcools primaires ou secondaires en aldéhydes ou cétones est généralement la température d'ébullition du mélange réactionnel. Lorsque l'eau formée par la réaction est éliminée par distillation azéotropique, cette élimination peut se faire de manière continue ou discontinue. L'eau de l'azéotrope binaire eau-solvant organique peut être éliminée, soit par passage dudit azéotrope sur un solide adsorbant l'eau tel que les tamis moléculaires avant recyclage du solvant organique, soit par décantation. Après décantation de la couche de l'azéotrope hétérogène contenant la majeure partie de l'eau on peut recycler l'autre couche dans le mélange réactionnel.

La concentration de l'alcool à déshydrogéner dans le mélange réactionnel n'est pas critique. Habituellement l'alccol mis en oeuvre représente de 1 a 60 % en poids du mélange réactionnel et de préférence de 2 à 30 % en poids.

L'élimination de l'eau formée lors de la réaction de déshydrogénation de l'alccol de formule (I) permet, notamment lorsque l'on opère avec des concentrations importantes en alcool, de conserver une cinétique de réaction plus élevée qu'en cas de maintien de cette eau dans le milieu réactionnel.

L'oxygène utilisé dans le procédé selon l'invention peut être de l'oxygène moléculaire de l'air, de l'air enrichi ou appauvri en oxygène ou tout autre mélange de l'oxygène avec un gaz inerte.

La pression totale sous laquelle s'effectue la réaction peut être supérieure, égale ou inférieure à la pression atmosphérique elle est généralement comprise entre 0,5 et 5 bars. La pression partielle d'oxygène est de préférence comprise entre 0,05 bar et 2 bars.

On peut opérer la déshydrogénation de l'alccol soit en maintenant une pression constante, soit en faisant circuler l'oxygène ou le gaz en contenant dans l'appereil où s'effectue la réacticn soit encore en faisant barboter l'oxygène ou le gaz en contenant dans le mélange réactionnel.

L'appareillage dans lequel le procédé selon l'invention est mis en oeuvre n'est pas spécifique audit procédé.

5

Les exemples qui suivent ont pour but d'illustrer l'invention sans en limiter la portée.

**Exemple 1 et Essai compatatif 1A**

On utilise un ballon de 250 cm³ comportant un agitateur central à ailettes, une colonne Vigreux azéotropique avec un réfrigérant ascendant, un tube plongeant d'arrivé de l'oxygène ou de l'air d'un thermomètre et d'un système de chauffage électrique.

La colonne azéotropique permet la décantation de l'azéotrope:
- la couche riche en eau est éliminée
- la couche contenant le solvant est recyclée.

On charge les réactifs suivants:

- n-octanol : 13,0 g (0,1 mole)
- n-hexane : quantité suffisante pour (qsp) : 100 cm³
- Pt - Bi/C à 5 % en poids de Pt par rapport à (Pt + C)
(rapport molaire Bi/Pt = 0,34) : 5,46 g

Le catalyseur Pt - Bi/noir de carbone est préparé de la manière suivante.

Dans 360 cm³ d'eau on place 49,5 g de noir de carbone 2S et 13,7 g de $Na_2 CO_3$ (solution (I). On prépare une solution (II) d'acide chloroplatinigue (6,32 g comptés à 37,6 % de Pt en poids) et de $BiCl_3$ (1,15 g) dans 10 cm³ d'HCl 2N. Cette soluticn (II) est coulée lentement dans la solution (I) en agitant. On chauffe environ 1h vers 95°C. Après refroidissement à 75°C on coule 14,2 cm³ d'une solution aqueuse de formol (à 35 % en poids par volume).

On chauffe ensuite 30 min environ vers 95°C. Après refroidissement le catalyseur obtenu est filtré, lavé per 1,4 l d'eau et séché sous vide (133 pascals à 40° jusqu'à poids constant.

On agite à 700 tours/minute environ et on chauffe à reflux (environ 70°C) en faisant barboter de l'air avec un débit de 7 litres/heure.

L'eau est éliminée per distillation azéotropique.

Des prélèvements du mélange réactionnel sont effectués pour différentes durées de maintien en température.

On détermine par dosage en chromatographie en phase gazeuse le taux de transformation de l'octanol (TT) et les rendements (T en moles/moles %) en octanal en acide octanoïque, en ester (octanoate d'octyle et en acétal formé par l'octanal et l'octanol.

Les TT et RT sont calculés de 2 façons:
1) - le TT tient compte de l'alcool disparu gu'il soit transformé en aldéde, en acide en ester (1 acide + 1 alcool) ou en acétal (1 aldéhyde + 2 alcools)
- les RT sont donc établis par rapport à l'alcool disparu et les calculs pour l'ester et l'acétal sont les suivants:

$$\text{RT ester} = \frac{\text{nombre de moles d'alccol formé}}{\text{nombre de moles d'alcool disparu}} \times 2 \times 100$$

$$\text{RT acétal} = \frac{\text{nombre de moles d'acétal formé}}{\text{nombre de moles d'alcool disparu}} \times 3 \times 100$$

2 Le TT ne tient compte que de l'alccol réellement "oxydé" (en aldéhyde ou en acide ou en produits secondaires) mais non de l'alccol combiné sous forme d'ester ou d'acétal.

Alcool "oxydé" = (nombre de moles d'alcool disparu)(nombre de moles d'ester) - 2 (nombre de moles d'acétal)

Les RT sont calculés de la façon suivante:

$$\text{RT aldéhyde} = \frac{\text{moles d'aldéhyde libre + moles d'acetal}}{\text{moles d'alcool oxydé}} \times 100$$

$$\text{RT acide} = \frac{\text{moles d'acide libre + moles d'ester}}{\text{moles d'alcool oxydé}} \times 100$$

Le tableau I ci-après rassemble les différents résultats.

Un essai comparatif est effectué dans les mêmes conditions que l'exemple 1, mais sans élimination de l'eau formée; le milieu éactionnel est maintenu à reflux total pendant 2 heures. Le tableau I bis rassemble les

résultats obtenus: les TT et RT sont exprimés selon le mode de calcul (1) par rapport à l'alccol disparu).

| Durée à 70°C | TT % alcool | RT % aldéhyde | RT % acide | RT % ester | RT % acétal |
|---|---|---|---|---|---|
| 35 min | (1) 27 | 63,5 | 4,2 | 18,0 | 13,5 |
|  | (2) 21,5 | 83,5 | 16,3 | — | — |
| 1 heure | (1) 42,0 | 69,5 | 4,5 | 20,5 | 5,0 |
|  | (2) 36,5 | 82,5 | 17,0 | — | — |
| 2 heures | (1) 72,0 | 63,0 | 6,0 | 23,5 | 3,0 |
|  | (2) 62,5 | 73,5 | 20,5 | — | — |
| 2h 55 min | (1) 90,0 | 58,5 | 15,5 | 26,0 | 0 |
|  | (2) 78,0 | 67,5 | 32,5 | — | — |

**Tableu I**

| Durée à 70°C | TT % alcool | RT % aldéhyde | RT % acide | RT % ester | RT % acétal |
|---|---|---|---|---|---|
| 2 heures | 49,5 | 58,5 | 18 | 16,5 | 0 |

**Tableu I bis (essai oomparatif 1 A)**

Exemple 2
On répète l'exemple 1 avec les mêmes quantités de réactifs en opérant pendant une durée de 2 heures 30 min selon le même mode opératoire.

Après cette première opération on lave le catalyseur à l'hexane et on répète à nouveau l'essai avec ce catalyseur recyclé et une nouvelle charge de réactifs.

Les résultats sont rassemblés dans le tableau II (les TT et RT sont exprimés selon le mode calcul (1) par rapport à l'alccol disparu)

| Essai | TT % alcool | RT % aldéhyde | RT % acide | RT % ester | somme des RT % |
|---|---|---|---|---|---|
| Exemple 2 A | 86 | 63,1 | 9,0 | 21,2 | 93,3 |
| Exemple 2 B avec catalyseur recyclé | 81,6 | 71,4 | 9,9 | 16,0 | 97,3 |

**Tableau II**

Le bilan (somme des RT) gui est généralement légèrement déficitaire devient pratiquement quantitatif lors de l'essai de recyclage.

Exemple 3 et 4
Les exemples 3 et 4 sont effectués dans le même appareillage que l'exemple 1, avec les mêmes réactifs et selon le même mode opératoire, mais avec soit de l'oxygène moléculaire pur, soit avec de l'air dilué contenant 12 % en volume d'oxygenène L'exemple 1 (fin d'essai) est rappelé à titre de comparaison. Les résultats sont rassemblés dans le tableau III. Les TT % et RT % sont déterminés selon le calcul (1) c'est-à-dire en considérant l'octanol disparu.

| Exemples | Oxydant | Durée | TT % alcool | RT % aldéhyde | RT % acide | RT % ester |
|---|---|---|---|---|---|---|
| Exemple 1 | Air à 21 % d'oxygène | 2h 55 | 90,0 | 58,5 | 15,5 | 26,0 |
| Exemple 3 | Oxygène pur | 1h 35 | 91,5 | 42,0 | 39,0 | 11 |
| Exemple 4 | Air à 12 % d'oxygène | 2h | 62,5 | 64,3 | 4,3 | 18,2 |

**Tableau III**

Exemples 5 à 7

Ces exemples sont effectués dans le même appareillage que l'exemple 1, avec les mêmes réactifs et selon le même mode opératoire, à l'exception du solvant utilisé et par conséquent de la température du mélange réactionnel, et dans le cas de l'acétate d'éthyle du mode d'élimination de l'eau : l'azéotrope binaire eau-acétate d'éthyle est passé sur un tamis moléculaire qui adsorbe l'eau et l'acétate d'éthyle est renvoyé dans le mélange réactionnel. La durée de maintien à la température de réaction est de 2 heures. Les résultats [TT % et RT % sont déterminés selon le mode de calcul (1) c'est à dire en considérant l'octanol disparu] sont rassemblés dans le tableau IV.

| Exemples | solvant | Température | TT % alcool | RT % aldéhyde | RT % acide | RT % ester | sélectivité* |
|---|---|---|---|---|---|---|---|
| Exemple 5 | acétate d'éthyle | 70°C | 76,4 | 70,3 | 15,0 | 7,0 | 0,79 |
| Exemple 6 | diméthoxy éthane | 79°C | 67,0 | 62,4 | 28,9 | 5,0 | 0,66 |
| Exemple 7 | oxyde de diisopropyle | 68°C | 67,4 | 64,0 | 6,5 | 21,0 | 0,79 |

$$* \text{ sélectivité en aldéhyde} = \frac{\text{moles d'aldéhyde formées}}{\text{total moles d'aldéhyde, d'acide et d'ester formées}}$$

TABLEAU IV

**Tableau IV**

Exemple 8

L'essai est effectué avec le même appareillage que dans l'exemple 1 et selon le même mode opératoire. Les reactifs sont les suivants:

- n-propanol          : 2,4 g (0,04 mole)
- n-hexane             : qsp 100 cm$^3$
- Pt-Bi/C à 5 % de Pt   : 2,18 g

(cf exemple 1)

Le rapport molaire Pt/propanol est de 1,4 %
La température du mélange réactionnel est de 70°C environ et elle maintenue pendant 2 heures.
L'oxydant utilisé est l'oxygène moléculaire avec un débit de 6 litres/heure.
On obtient les résultats suivants (déterminés selon le calcul (1) c'est à dire par rapport à l'alccol disparu).

TT du propanol : 29,0 %
RT en propanal : 70,0 %

Exemples 9 à 11
Les essais sont effectués avec le même appareillage gue dans l'exemple 1 et selon le même mode opéatoire mais en mettant en oeuvre des alccols différents (0,1 mole).
Les TT et RT sont déterminés selon le calcul (1), c'est à dire par rapport à l'alccol disparu.
Les résultats sont rassemblés dans le tableau V.

| Exemples | alcools mis en oeuvre | TT % alcool | RT % en aldéhyde ou cétone |
|---|---|---|---|
| Exemple 9 | Octanol-2 | 96,0 % | 99,0 % |
| Exemple 10 | Paratertiobutyl-cyclohexanol | 66,0 % | 98,0 % |
| Exemple 11 | Méthyl-3 butène-2 ol-1 (prénol) | 99,0 % | 81,0 % |

**Tableau V**

Exemple 12
L'essai est effectué avec le même appareillage que dans l'exemple 1 et selon le même mode opératoire.
Les réactifs suivants sont chargés:

- propanediol-1,2 : 8,1 g
- acetate d'éthyle : qsp 100 cm$^3$
- Pt-Bi/C à 5 % de Pt : 5,84 g

(cf exemple 1)

La vitesse d'agitation est de 700 tours/minute.
L'oxydant est de l'air qui est injecté avec un débit de 8 litres/heure environ.
Les réesultats, déterminés selon le calcul (1) (alcool disparu), sont les suivants:
Après 2 heures de réaction : TT = 45 % RT en hydroxyacétone : 98 %
Après 4h 15 de réaction : TT = 60 % RT en hydroxyacétone : 84,5 %
Exemple 13
L'essai est effectué avec le même appareillage que dans l'exemple 1 et selon le même mode opératoire.
Les réactifs suivants sont chargés:

- méthyl-3 butèe-3 ol-1 : 8,6 g (0,1 mole)
- acétate de butyle : qsp 100 cm$^3$
- Pt-Bi/C à 5 % en poids de Pt : 6,4 g

(cf exemple 1)

La vitesse d'agitation est de 700 tours/minute.

La température à laquelle est maintenu le mélange réactionnel est de 125°C (reflux).

L'oxydant est de l'air qui est injecté avec un débit de 7 litres/heure environ.

Après 4 heures de réaction on obtient les résultats suivants (déterminés selon le mode de calcul (1), c'est à dire en considén l'alcool disparu):

TT du méthyl -3 butène-3 ol-1      : 89 %
RT en méyhyl-3 butène-2 al-1      : 55 %

(isomerisation en cours d'oxydation)

Exemple 14

L'essai est effectué avec le même appareillage que dans l'exemple 1 selon le même mode opératoire et avec les mêmes réactifs, mais avec un catalyseur Rh-Bi/C à 5 % en poids de Rh par rapport au total (Rh + C) et avec un rapport molaire Bi/Rh de 0,34.

On obtient les résultats suivants après un maintien de 2 heures en température (70°C). A titre de comparaison un essai identique a été effectué avec un catalyseur Rh/C à 5 % en poids de Rh.

- Avec Rh-Bi/C      - TT du n-cctanol      : 6 %
                   - RT en octanal      : 40 %
- Avec Rh/C      - TT du n-octanol      : 7 %
                   - RT en octanal      : 16 %

Il n'a pas été détecté d'acide octanoïque d'octanoate d'octyle ni d'acétal.

Exemple 15

L'essai est effectué avec le même appareillage que dans l'exemple 1 selon le même mode opératoire et avec les mêmes réactifs à l'exception:

- du solvant : le n-octane remplace le n-hexane
- du catalyseur : Ru-Bi/C à 5 % en poids de Ru par rapport à l' ensemble (Ru + C) remplace Pt-Bi/C.

La température à laquelle est opérée la réaction est de 125°C environ et l'essai est maintenu 2 heures à cette température.

On obtient les résultats suivants (déterminés selon le mode de calcul (1).

- TT du n-octanol      : 38 %
- RT en octanal      : 4 %
- RT en acétal      : 52 %
(dicctylacétal de l'octanal)
- RT en acide octanoïque      : 0 %
- RT en octanoate d'octyle      : 1 %

Exemple 16 à 18

Les essais sont effectués avec l'appareillage et selon le mode opératorie décrits dans l'exemple 1.

Les réactifs sont les mêmes gue dans l'exemple 1 et dans les mêmes proportions relatives, mais le catalyseur utilisé varie selon les exemples : Pt-Bi/C, Pt-Pb/C, Pt-Cd/C.

Les rapports metal noble/support et cocatalyseur/metal noble sont les mêmes que dans l'exemple 1.

La durée du maintien en température (70°C) est de 2 heures dans chaque essai.

A titre de comparaison un essai a été effectué avec du Pt/C sans cocatalyseur.

Les résultats (déterminés selon le mode de calcul (1) sont rassemblés dans le tableau VI.

10     .

| Essais | catalyseur | TT % des n-octanol | RT % en octanal | RT % en acétal | RT % acide | RT % ester | somme des RT % |
|---|---|---|---|---|---|---|---|
| Essai comparatif | Pt/C | 21,0 % | 17,2 % | 7,6 % | 3,2 % | 1,3 % | 29,3 % |
| Exemple 16 | Pt-Bi/C | 89,6 % | 58,8 % | 0 | 7,8 % | 19,8 % | 86,4 % |
| Exemple 17 | Pt-Pb/C | 26,8 % | 56,7 % | 0 | 9,5 % | 2,0 % | 68,2 % |
| Exemple 18 | Pt-Cd/C | 10,8 % | 48,1 % | 0 | 8,6 % | 0 | 56,7 % |

**Tableau VI**

**Revendications**

1) Procédé de préparation d'aldéhydes ou de cétones par oxydation catalytique à l'aide d'oxygène moléculaire ou d'un gaz en contenant, en milieu liquide des alcools primaires ou secondaires de formule générale (I):

$R_2$ - CHOH - $R_1$ (I)

dans laguelle:

- $R_1$ et $R_2$, identigues ou différents représentent:

+ un radical alkyle linéaire ou ramifié ayant de 1 à 23 atomes de carbone;

+ un radical alkényle ou alkynyle linéaire ou ramifié ayant de 3 à 23 atomes de carbone et dont la double ou la triple liaison n'est pas située sur l'atome de carbone directement lié à l'atome de carbone portant la fonction OH,

+ un radical alkadiényle ou alkadiynyle, linéaire ou ramifié, ayant de 5 à 23 atomes de carbone et dont l'atome de carbone directement lié à l'atome de carbone portant la fonction OH ne comporte pas de liaison multiple;

+ un radical cycloaliphatique ayant de 3 à 12 atomes de carbone;

+ un radical phénylalkyle dont la partie alkyle comporte 1 à 14 atomes de carbone;

les différents radicaux indiqués précédemment pouvant comporter un ou plusieurs substituants tels que groupement hydroxyle, groupement alkoxy ayant de 1 à 4 atomes de carbone, groupement nitro, groupement nitrile ou atome d'halogène,

- $R_1$ ou $R_2$ représente un atome d'hydrogène

- $R_1$ et $R_2$ peuvent former ensemble un radical alkylène linéaire ou ramifié, ayant de 4 à 23 atomes de carbone et ayant ou non un ou plusieurs substituants tels que groupement hydroxyle, groupement alkoxy ayant de 1 à 4 atomes de carbone, groupement nitro groupement nitrile, ou atome d'halogène,

- $R_1$ et $R_2$ isolément ou ensemble, sont constitués par un enchaînement de plusieurs radicaux choisis parmi les radicaux, que peuvent représenter $R_1$ et $R_2$, indiqués précédemment,

- $R_1$ et $R_2$ sont tels que la formule (I) comporte au plus 36 atomes de carbone;

caractérisé en ce que:

- le catalyseur est constitué par un métal noble du groupe VIII de la classification périodique des éléments associé à un métal choisi dans le groupe comprenant le bismuth, le plomb, le cadmium, le mercure, l'indium, le tellure, l'étain, l'argent et leurs dérivés, déposés sur un même support solide,

- le milieu liquide est constitué par un solvant organique,

- la température de réaction est comprise entre 60°C et 180°C,

- l'on élimine l'eau formée par distillation azéotropique.

2) Procédé selon 1a revendications 1 caractérisé en ce que les alcools auxquels il s'applique sont des alcools primaires répondant à la formule générale (I):

$R_2$ - CHOH - $R_1$ (I)

dans laquelle:

- $R_1$ est un atome d'hydrogène,

- $R_2$ représente:

+ un radical alkyle linéaire ou ramifié ayant de 1 à 23 atomes de carbone;

+ un radical alkényle ou alkynyle linéaire ou ramifié ayant de 3 à 23 atomes de carbone, et dont la double ou la triple liaison n'est pas située sur l'atome de carbone directement lié à l'atome de carbone portant la fonction OH,

+ un radical alkadiényle ou alkadiynyle, linéaire ou ramifié, ayant de 5 à 23 atomes de carbone et dont l'atome de carbone directement lié à l'atome de carbone portant la fonction OH ne comporte pas de liaison multiple;

+ un radical cycloaliphatigue ayant de 3 à 12 atomes de carbone;

+ un radical phénylalkyle dont la partie alkyle ccmporte 1 à 14 atomes de carbone;

les différents radicaux indiqués précédemment pouvant comporter un ou plusieurs substituants tels que

**0 112 261**

groupement hydroxyle, groupement alkoxy ayant de 1 à 4 atomes de carbone, groupement nitro, groupement nitrile ou atome d'halogène,
- $R_2$ est constitué par un enchaînement de plusieurs radicaux choisis parmi les radicaux que peut représenter $R_1$, indiqués précédemment,
- $R_2$ est tel que la formule (I) ccmporte au plus 36 atomes de carbone.

3) Procédé selon l'une quelconque des revendications 1 à 2 caractérisé en ce que le catalyseur utilisé est constitué par l'association
- d'un métal noble choisi parmi le platine, le palladium, le ruthénium et le rhodium,
- d'un cocatalyseur choisi parmi le bismuth, le plomb et le cadmium déposés sur un même support inerte tel que le noir de carbone, la silice, la silice-alumine, l'alumine, la magnésie et le sulfate de baryum.

4) Procédé selon l'une des revendications 1 à 3 caractérisé en ce que dans le catalyseur, le rapport molaire cocatalyseur/métal noble est compris entre 0,05 et 10 et de préférence entre 0,1 et 2.

5) Procédé selon l'une des revendications 1 à 4 caractérisé en ce que le catalyseur est constitué par l'association du platine et d'un cocatalyseur choisi parmi le bismuth, le plomb et le cadmium déposés sur du noir de carbone.

6) Procédé selon l'une quelconque des revendications 1 à 5, caractérisé en ce que l'eau formée au cours de la réaction est éliminée par distillation azéotropique à l'aide d'un solvant organique choisi de telle façon que l'azéotrope binaire qu'il forme avec l'eau ait un point d'ebullition
- d'au moins 50°C,
- inférieur au point d'ébullition de l'alcool à déshydrogéner,
- inférieur au point d'ébullition de l'azéotrope binaire que ledit alcool serait susceptible de former avec l'eau ou avec le solvant organigue lui-même.

7) Procédé selon l'une des revendications 1 à 6 caractérisé en ce que le solvant organique a un point d'ébullition compris entre 50°C et 200°C et est choisi parmi les hydrocarbures aliphatiques, cycloaliphatiques ou aromatiques; les esters d'alkyle ou d'alkényle d'acides carboxyliques aliphatiques; les éthers aliphatiques; aromatiques ou cycliques; les nitriles aliphatiques; cycloaliphatiques ou aromatiques, les cétones aliphatiques, cycloaliphatiques ou aromatiques.

8) Procédé selon l'une des revendications 1 à 7, caractérisé en ce que l'azéotrope binaire eau-solvant organique est hétércqène et qu'après décantation de la couche riche en eau, le solvant organique est recyclé dans le mélange réactionnel.

9) Procédé selon l'une quelconque des revendications 1 à 8, caractérisé en ce que l'oxydation est faite à l'aide d'oxygène d'air enrichi ou appauvri en oxygène ou d'un autre mélange d'oxygène avec un gaz inerte, sous une pression totale comprise entre 0,5 et 5 bars et une pression partielle d'oxygène comprise entre 0,05 et 2 bars.

10) Procédé selon l'une quelconque des revendications 1 à 9, caractérisé en ce que le rapport pondéral métal noble du catalyseur/alcool est compris entre 0,01 et 10 % et de préférence entre 0,1 et 5 %.

**Claims**

1. Process for the preparation of aldehydes or of ketones by catalytic oxidation using molecular oxygen or a gas containing it, in a liquid medium, of primary or secondary alcohols of general formula (I):
$$R_2 - CHOH - R_1 \text{ (I)}$$
n which:
- $R_1$ and $R_2$, which are identical or different, denote:
- a straight-chain or branched alkyl radical containing from 1 to 23 carbon atoms,
- a straight-chain or branched alkenyl or alkynyl radical containing from 3 to 23 carbon atoms and in which the double or triple bond is not situated on the carbon atom bonded directly to the carbon atom bearing the OH group,
- a straight-chain or branched alkadienyl or alkadiynyl radical containing from 5 to 23 carbon atoms and in which the carbon atom directly bonded to the carbon atom bearing the OH group does not comprise a multiple bond,
- an alicyclic radical containing from 3 to 12 carbon atoms,
- a phenylalkyl radical in which the alkyl moiety comprises 1 to 14 carbon atoms,
it being possible for the various radicals indicated above to comprise one or more substituents such as a hydroxyl group, an alkoxy group containing from 1 to 4 carbon atoms, a nitro group, a nitrile group or a halogen atom,
- $R_1$ or $R_2$ denotes a hydrogen atom,
- $R_1$ and $R_2$ may together form a straight-chain or branched alkylene radical containing from 4 to 23 carbon atoms and having or not having one or more substituents such as a hydroxyl group, an alkoxy group containing from 1 to 4 carbon atoms, a nitro group, a nitrile group or a halogen atom,
- $R_1$ and $R_2$, separately or together, consist of a concatenation of several radicals chosen from the radicals which may be denoted by $R_1$ and $R_2$, which are indicated above,
- $R_1$ and $R_2$ are such that the formula (I) comprises at most 36 carbon atoms;

12

characterized in that:

- the catalyst consists of a noble metal of Group VIII of the Periodic Classification of the elements associated with a metal chosen from the group comprising bismuth, lead, cadmium, mercury, indium, tellurium, tin, silver and their derivatives, which are deposited on the same single solid support,
- the liquid medium consists of an organic solvent,
- the reaction temperature is between 60°C and 180°C, and
- the water formed is removed by azeotropic distillation.

2. Process according to Claim 1, characterized in that the alcohols to which it applies are primary alcohols corresponding to the general formula (I):

$R_2 - CHOH - R_1$ (I)

in which.:
- $R_1$ is a hydrogen atom,
- $R_2$ denotes:
- a straight-chain or branched alkyl radical containing from 1 to 23 carbon atoms;
- a straight-chain or branched alkenyl or alkynyl radical containing from 3 to 23 carbon atoms and in which the double or the triple bond is not situated on the carbon atom bonded directly to the carbon atom bearing the OH group,
- a straight-chain or branched alkadienyl or alkadiynyl radical containing from 5 to 23 carbon atoms and in which the carbon atom bonded directly to the carbon atom bearing the OH group does not comprise a multiple bond,
- an alicyclic radical containing from 3 to 12 carbon atoms, and
- a phenylalkyl radical in which the alkyl moiety comprises 1 to 14 carbon atoms;

it being possible for the various radicals indicated above to comprise one or more substituents such as a hydroxyl group, an alkoxy group containing from 1 to 4 carbon atoms, a nitro group, a nitrile group or a halogen atom,
- $R_2$ consists of a concatenation of several radicals chosen from the radicals which may be denoted by $R_1$, which are indicated above,
- $R_2$ is such that the formula (I) comprises at most 36 carbon atoms.

3. Process according to either of Claims 1 and 2, characterized in that the catalyst used consists of the association
- of a noble metal chosen from platinum, palladium, ruthenium and rhodium, and
- of a cocatalyst chosen from bismuth, lead and cadmium,
- which are deposited on the same single inert support such as carbon black, silica, silica-alumina, alumina, magnesia and barium sulphate.

4. Process according to any one of Claims 1 to 3, characterized in that, in the catalyst, the molar ratio cocatalyst/noble metal is between 0.05 and 10 and preferably between 0.1 and 2.

5. Process according to one of Claims 1 to 4, characterized in that the catalyst consists of the association of platinum and of a cocatalyst chosen from bismuth, lead and cadmium, which are deposited on carbon black.

6. Process according to any one of Claims 1 to 5, characterized in that the water formed during the reaction is removed by azeotropic distillation with the aid of an organic solvent chosen so that the binary azeotrope which it forms with water has a boiling point
- of at least 50°C,
- below the boiling point of the alcohol to be dehydrogenated, and
- below the boiling point of the binary azeotrope which the said alcohol would be capable of forming with water or with the organic solvent itself.

7. Process according to one of Claims 1 to 6, characterized in that the organic solvent has a boiling point between 50°C and 200°C and is chosen from aliphatic, alicyclic or aromatic hydrocarbons, alkyl or alkenyl esters of aliphatic carboxylic acids, aliphatic, aromatic or cyclic ethers, aliphatic, alicyclic or aromatic nitriles and aliphatic, alicyclic or aromatic ketones.

8. Process according to one of Claims 1 to 7, characterized in that the water-organic solvent binary azeotrope is heterogeneous and that, after phase separation of the water-rich layer, the organic solvent is recycled into the reaction mixture.

9. Process according to any one of Claims 1 to 8, characterized in that the oxidation is performed using oxygen, oxygen-enriched or depleted air or another mixture of oxygen with an inert gas, under a total pressure of between 0.5 and 5 bars and a partial pressure of oxygen of between 0.05 and 2 bars.

10. Process according to any one of Claims 1 to 9, characterized in that the weight ratio of the noble metal of the catalyst to the alcohol is between 0.01 and 10% and preferably between 0.1 and 5%.

**Patentansprüche**

1. Verfahren zur Herstellung von Aldehyden oder Ketonen in flüssigem Milieu mittels molekularem Sauerstoff oder einem diesen enthaltenden Gas durch katalytische Oxydation primärer oder sekundärer Alkohole der allgemeinen Formel (I):

13      .

$R_2$ - CHOH - $R_1$ (I)
bei der:

- $R_1$ und $R_2$ identisch sind oder verschieden und darstellen:

+ einen linearen oder verzweigten Alkylrest mit 1 bis 23 Kohlenstoffatomen;

+ einen linearen oder verzweigten Alkenyloder Alkinylrest mit 3 bis 23 Kohlenstoffatomen, bei dem die Doppel- oder die Dreifachbindung sich nicht an dem Kohlenstoffatom befindet, das direkt verknüpft ist mit dem Kohlenstoffatom, das die OH-Funktion trägt;

+ einen Alkadienyl oder Alkadiinylrest, linear oder verzweigt, mit 5 bis 23 Kohlenstoffatomen, bei dem das Kohlenstoffatom, das direkt mit dem Kohlenstoffatom verknüpft ist, das die OH-Funktion trägt, keine Mehrfachbindung aufweist;

+ einen cycloaliphatischen Rest mit 3 bis 12 Kohlenstoffatomen;

+ einen Phenylalkylrest, bei dem der Alkylteil 1 bis 14 Kohlenstoffatome aufweist; die verschiedenen vorgenannten Reste können einen oder mehrere Substituenten aufweisen wie Hydroxylgruppen, Alkoxygruppen mit 1 bis 4 Kohlenstoffatomen, Nitrogruppen, Nitrilgruppen oder Halogenatome,

- $R_1$ oder $R_2$ ein Wasserstoffatom darstellen

- $R_1$ und $R_2$ gemeinsam einen linearen oder verzweigten Alkylenrest bilden können, mit 4 bis 23 Kohlenstoffatomen ohne Substituenten oder mit einem oder mehreren Substituenten wie Hydroxylgruppen, Alkoxylgruppen mit 1 bis 4 Kohlenstoffatomen, Nitrogruppen, Nitrilgruppen oder Halogenatomen,

- $R_1$ und $R_2$ isoliert oder gemeinsam, aus einer Aneinanderreihung mehrerer Reste bestehen, ausgewählt unter den vorgenannten Resten, die für $R_1$ und $R_2$ in Frage kommen,

- $R_1$ und $R_2$ gemäß der Formel (I) höchstens 36 Kohlenstoffatome aufweisen;

dadurch gekennzeichnet, daß

- der Katalysator besteht aus einem Edelmetall der Gruppe VIII des Periodensystems in Verbindung mit einem Metall, ausgewählt aus der Gruppe umfassend Wismut, Blei, Cadmium, Quecksilber, Indium, Tellur, Zinn, Silber und deren Derivate, angeordnet auf dem gleichen festen Träger,

- das flüssige Milieu aus einem organischen Solvens besteht,

- die Temperatur der Reaktion zwischen 60°C und 180°C liegt,

- das gebildete Wasser durch azeotrope Destillation entfernt wird.

2. Verfahren nach Anspruch 1, dadurch gekennzeichnet, daß die Alkohole, auf die es angewandt wird, primäre Alkohole entsprechend der allgemeinen Formel (I) sind:

$R_2$ - CHOH - $R_1$ (I)

in der:

- $R_1$ ein Wasserstoffatom ist,

- $R_2$ darstellt:

+ einen linearen oder verzweigten Alkylrest mit 1 bis 23 Kohlenstoffatomen;

+ einen linearen oder verzweigten Alkenyl- oder Alkinylrest mit 3 bis 23 Kohlenstoffatomen, bei dem die Doppel- oder die Dreifachbindung sich nicht an einem Kohlenstoffatom befindet, das direkt mit einem Kohlenstoffatom verknüpft ist, das eine OH-Funktion trägt,

+ einen linearen oder verzweigten Alkydienyl- oder Alkinylrest, mit 5 bis 23 Kohlenstoffatomen, bei dem das Kohlenstoffatom, das direkt mit dem Kohlenstoffatom verknüpft ist, das die OH-Funktion trägt, keine Mehrfachbindung aufweist;

+ einen cycloaliphatischen Rest mit 3 bis 12 Kohlenstoffatomen;

+ einen Phenylalkylrest bei dem der Alkylteil aus 1 bis 14 Kohlenstoffatomen besteht;

die verschiedenen vorgenannten Rest einen oder mehrere Substituenten aufweisen können, sowie Hydroxylgruppen, Alkoxygruppen mit 1 bis 4 Kohlenstoffatomen, Nitrogruppen, Nitrilgruppen oder Halogenatome,

- $R_2$ aus einer Aneinanderreihung mehrerer Rest besteht, ausgewählt unter den vorgenannten Resten, die für $R_1$ in Frage kommen,

- $R_2$ gemäß der Formel (I) höchstens 36 Kohlenstoffatome aufweist.

3. Verfahren nach einem der Ansprüche 1 bis 2, dadurch gekennzeichnet, daß der verwendete Katalysator aus einem Gemisch besteht

- eines Edelmetalls ausgewählt zwischen Platin, Palladium, Ruthenium und Rhodium,

- mit einem Cokatalysators ausgewählt zwischen Wismut, Blei und Cadmium, angeordnet auf dem gleichen inerten Träger wie Kohle, Siliziumoxid, Siliziumoxid-Aluminiumoxid, Aluminiumoxid, Magnesiumoxid und Bariumsulfat.

4. Verfahren nach einem der Ansprüche 1 bis 3, dadurch gekennzeichnet, daß bei dem Katalysator das molare Verhältnis Cokatalysator/Edelmetall zwischen 0,05 und 10, vorzugsweise zwischen 0,1 und 2 liegt.

5. Verfahren nach einem der Ansprüche 1 bis 4, dadurch gekennzeichnet, daß der Katalysator besteht aus einem Gemisch von Platin und einem Cokatalysator ausgewählt zwischen Wismut, Blei und Cadmium, aufgebracht auf Kohle.

6. Verfahren nach einem der Ansprüche 1 bis 5, dadurch gekennzeichnet, daß das im Verlauf der Reaktion gebildete Wasser durch azeotrope Destillation entfernt wird mit Hilfe eines organischen Solvens, ausgewählt in der Weise, daß das binäre Azeotrop, das dieses mit Wasser bildet, einen Siedepunkt hat

- von mindestens 50°C,

- unterhalb des Siedepunkts des zu dehydrogenierenden Alkohols,

- unterhalb des Siedepunkts des binären Azeotrops, das besagter Alkohol mit Wasser oder dem organischen Solvens selbst bilden könnte.

7. Verfahren nach einem der Ansprüche 1 bis 6, dadurch gekennzeichnet, daß das organische Solvens einen Siedepunkt zwischen 50°C und 200°C hat und ausgewählt wird zwischen den aliphatischen, cycloaliphatischen oder aromatischen Kohlenwasserstoffen, den aliphatischen, aromatischen oder cyclischen Äthern, den aliphatischen, cycloaliphatischen oder aromatischen Nitrilen, den aliphatischen, cycloaliphatischen oder aromatischen Ketonen.

8. Verfahren nach einem der Ansprüche 1 bis 7, dadurch gekennzeichnet, daß das binäre Azeotrop Wasserorganisches Solvens heterogen ist und das nach der Absetzung der wasserreichen Phase das organische Solvens in die Reaktionsmischung zurückgeführt wird.

9. Verfahren nach einem der Ansprüche 1 bis 8, dadurch gekennzeichnet, daß die Oxidation ausgeführt wird mittels Sauerstoff, mit Sauerstoff angereicherter Luft oder sauerstoffarmer Luft oder mit einer Mischung von Sauerstoff mit einem inerten Gas, bei einem Gesamtdruck zwischen 0,5 und 5 bar und einem Sauerstoffpartialdruck zwischen 0,05 und 2 bar.

10. Verfahren nach einem der Ansprüche 1 bis 9, dadurch gekennzeichnet, daß das Gewichtsverhältnis Edelmetall des Katalysators/Alkohol zwischen 0,01 und 10 % liegt, vorzugsweise zwischen 0,1 und 5 %.